# EUROPEAN PATENT APPLICATION

(11) **EP 4 696 273 A1**
(43) Date of publication of application: **18.02.2026**
(21) Application number: 24194091.5
(22) Date of filing: 12.08.2024
(51) Int. Cl.: A61B 34/30, A61M 25/01, A61M 25/09, A61B 90/00

(54) **ROBOTIC MODULE ASSEMBLY FOR AN ENDOVASCULAR PROCEDURE**

(71) Applicant: Koninklijke Philips N.V., 5656 AG Eindhoven (NL)
(72) Inventor: PATRICIU, Alexandru, Eindhoven (NL); BALICKI, Marcin Arkadiusz, Eindhoven (NL); HOFFMANN, Julia, Eindhoven (NL)
(74) Representative: Philips Intellectual Property & Standards

(57) **Abstract**

The invention relates to a robotic module assembly 2 for an endovascular procedure. The robotic module assembly 2 comprises a pathway 4 for accommodating a tubular device 14 for an endovascular procedure, a first drive roller 18 and a second drive roller 20. The robotic module assembly 2 further comprises a gear assembly 26 comprising a slot 28 that extends in a longitudinal direction 30 in parallel to the longitudinal axis 16 through the gear assembly 26. The slot 28 extends from an outer radial surface 32 of the gear assembly 26 to the pathway 4 along a trajectory 6. The trajectory 6 is at least partially curved. It has been found that by providing the slot 28 with a trajectory 6 which is at least partially curved, a specific sitting position of the tubular device 14 can be defined at the pathway 4.

## Description

### FIELD OF THE INVENTION

The invention relates to a robotic module assembly for an endovascular procedure, a method, a robotic apparatus and a use.

### BACKGROUND OF THE INVENTION

Robotic module assemblies are used for endovascular procedures to advance, retract and potentially rotate tubular devices, such as welding wires, needles, endovascular catheters, endovascular guidewires and the like. The translation of tubular devices is achieved by acting on the shaft of such devices.

In order to achieve reliable motion, the trajectory of the tubular a device must be constrained. This is important in order to prevent buckling of the tubular device within the robotic module assembly. Furthermore, it must be ensured that the device can be loaded and removed from the robotic module assembly conveniently. The tubular device is guided in the center of a robotic apparatus having a high mechanical complexity and comprising a number of gears. Loading the tubular device into and removing the tubular device from the robotic module assembly is thus challenging. In addition, it is required to ensure that the tubular device is in the right loaded position within the device.

From the prior art it is known to utilize a closable slot in a gear assembly to load the tubular device towards a pathway for accommodating the tubular device. However, the proposed device is rather complex and may not ensure that the motion of the tubular device is properly constrained.

### SUMMARY OF THE INVENTION

It is an object of the present invention to provide a robotic module assembly, a method, a robotic apparatus and a use ensuring that the motion of the tubular device is constrained when the tubular device is advanced or retracted, so as to avoid buckling of the tubular device. Furthermore, it is an object of the invention to ensure that the tubular device may be conveniently inserted and removed from the robotic assembly. It is yet another object of the invention to ensure that the tubular device is in the right loaded position within the device.

In a first aspect, a robotic module assembly for an endovascular procedure is presented. The robotic module assembly comprises a pathway for accommodating a tubular device for an endovascular procedure. The pathway extends along a longitudinal axis of the robotic module assembly. The robotic module assembly furthermore comprises a first drive roller arranged on a first side of the pathway and a second drive roller arranged on a second side of the pathway. The drive rollers are configured to advance and/or retract the tubular device. The robotic module assembly furthermore comprises a gear assembly. The gear assembly comprises a slot that extends in a longitudinal direction in parallel to the longitudinal axis through the gear assembly. The slot is configured to load a tubular device into the pathway. Moreover, the slot extends from an outer radial surface of the gear assembly of the pathway. The robotic module assembly furthermore comprises a loading assembly configured to modify a spacing between the first drive roller and the second drive roller between a loading state and a loaded state. The spacing of the drive rollers in the loading state leaves the pathway accessible via the slot. The spacing of the drive rollers in the loaded state prevents the pathway from being accessible via the slot.

It has been found that the loading assembly provides a reliable motion of the tubular device avoiding buckling, because the trajectory of the tubular device is constrained by and between the drive rollers. The loading assembly furthermore provides a mechanical closure mechanism which is reliable and easy to use, even when the drive rollers are arranged in the center of the robotic module assembly comprising the gear assembly. The loading assembly is compatible with a number of gear assembly designs comprising a slot. The slot may have a straight trajectory or a curved trajectory from the outer radial surface to the pathway.

In one embodiment, the spacing between the drive rollers is larger in the loading state than in the loaded state. In this way, the tubular device is constrained when the loading assembly is in the loaded state. In the loading state, however, the tubular device may be inserted between the drive rollers.

The tubular device may be engaged between the drive rollers in the loaded state in order to advance and/or retract the tubular device. By constraining the movement of the tubular device by means of the drive rollers, a reliable movement of the tubular device can be achieved.

In the loading state, the gap between the drive rollers ensures that the pathway is accessible via the slot. In the loaded state, the drive rollers are positioned such that the gap is closed preventing the pathway being accessible via the slot.

In one embodiment, the first drive roller is arranged opposite of the second drive roller in the loaded state. Thereby, the tubular device can be constrained by and between the drive rollers.

The loading assembly may be configured to pivot at least one of the drive rollers in order to modify the spacing between the drive rollers between the loading state and the loaded state. In one embodiment, the loading assembly comprises a motor in order to modify the spacing between the drive rollers between the loading state and the loaded state. Thereby, the loading and unloading of the tubular device may be at least partially or fully automated.

In one embodiment, a lever is provided which rotates a keyed shaft which supports the loading assembly in the loading position like a shim. An elastic member may be provided. In one embodiment the elastic member is arranged between the loading assembly and the keyed shaft. The elastic member may maintain the position with friction between the loading assembly and the keyed shaft. The lever may be actuated to rotate the keyed shaft into the loading position. In the loading position, the keyed shaft may no longer support the loading assembly. In the loading position, the elastic member rotates the loading assembly, and a gap is formed between the drive rollers. In one embodiment, the lever is connected to a motor. The motor may be configured to actuate the lever.

In one embodiment, the pathway constrains the tubular device along a Y-axis. The drive rollers may constrain the device along an X-axis which is perpendicular to the Y-axis.

In one embodiment, the loading assembly comprises a sitting profile configured to sit and/or accommodate the tubular device therein. The sitting profile is configured to accommodate the tubular device.

The sitting profile may match the end of the slot in the loading state. In one embodiment, the position of the sitting profile in the loading state is different from the position of the sitting profile in the loaded state. This may be achieved by providing a pivotable portion at the loading assembly. The sitting profile may be arranged in the pivotable portion. The sitting profile is transferred between the loading state and the loaded state by pivoting the pivotable portion about a pivot axis. Thereby, the tubular device may be constrained in the loaded state. The pivot axis is preferably spaced from the longitudinal axis of the robotic module assembly.

The pivotable portion may comprise a protruding edge extending adjacent to the slot. The protruding edge may enclose the sitting profile in the loaded state.

In one embodiment, the slot may comprise a bottom section. The bottom section is preferably aligned with the drive rollers.

In one embodiment, a lever is provided to move the loading assembly between the loading state and the loaded state. The loading assembly and the lever may comprise a linkage with two stable positions, namely the loading state and the loaded state. The lever may be actuated to slide the first drive roller or the second drive roller between the loading state and the loaded state. A rigid member may push the loading assembly into the loading state. The elastic member may hold the loading assembly in the loaded state.

In a second aspect, of the present invention, a robotic module assembly for an endovascular procedure is presented. The robotic module assembly comprises a pathway for accommodating a tubular device for an endovascular procedure. The pathway extends along a longitudinal axis of the robotic module assembly. The robotic module assembly furthermore comprises a first drive roller arranged on a first side of the pathway and a second drive roller arranged on a second side of the pathway. The drive rollers are configured to advance and/or retract the tubular device. The robotic module assembly furthermore comprises a gear assembly comprising a slot that extends in a longitudinal direction in parallel to the longitudinal axis through the gear assembly. The slot is configured to load a tubular device into the pathway. The slot extends from an outer radial surface of the gear assembly to the pathway along a trajectory. Preferably, the trajectory is at least partially curved.

It has been found that by providing a slot having a trajectory which is at least partially curved, a specific sitting position of the tubular device is defined at the pathway. In other words, by said slot trajectory being at least partially curved, the sitting of the device at the pathway is reinforced. All in all, the tubular device is fully constrained along the pathway by the set of partial constraints provided by the slot and the rollers.

In one embodiment, the slot comprises a transversal end portion aligned with the pathway. The transversal end portion extends substantially parallel to the longitudinal axis of the robotic module assembly. The end portion defines a sitting position for the tubular device at the pathway. In this way, an improved loading into the pathway for the tubular device is achieved.

In one embodiment, adjacent to the transversal end portion a transversal curved intermediate portion is arranged. The curved intermediate portion defines a turn of the tubular device. The tubular device passes the transversal curved intermediate portion before reaching the transversal end portion when the tubular device is inserted into the slot towards the pathway. By providing the transversal curved intermediate portion, the loading of the tubular device towards the pathway is further improved.

In a third aspect of the invention, a robotic module assembly for an endovascular procedure is provided. The robotic module assembly comprises a pathway for accommodating a tubular device for an endovascular procedure. The pathway extends along a longitudinal axis of the robotic module assembly. The robotic module assembly furthermore comprises a first drive roller arranged on a first side of the pathway and a second drive roller arranged on a second side of the pathway. The drive rollers are configured to advance and/or retract the tubular device. The robotic module assembly furthermore comprises a gear assembly comprising a slot. The slot extends in a longitudinal direction in parallel to the longitudinal axis through the gear assembly. The gear assembly comprises a first rotatable gear and a second rotatable gear. The gears are arranged substantially in parallel to each other. Each gear comprises the slot. The loading assembly may also comprise a slot. The slots are configured to load a tubular device into the pathway when the slots of the two gears are aligned in a loading angular position.

It has been found that by providing at least two gears having a slot, the slots of the gears need to be aligned in order to conveniently insert the tubular device into the robotic module assembly.

In one embodiment, the gear assembly comprises a sensing arrangement. The sensing arrangement may be configured to detect the loading angular position of the first rotatable gear and/or the second rotatable gear, and the loading assembly. By means of the sensing arrangement, it can be detected when the first rotatable gear and/or the second rotatable gear reaches the loading angular position.

Preferably, the sensing arrangement is configured to detect the loading angular position of the first rotatable gear and/or the second rotatable gear. This is beneficial, since both gears need to be in the loading angular position in order to load the tubular device.

In one embodiment, the sensing arrangement comprises a magnet attached to the first gear and/or the second gear and a magnet attached to the loading assembly. The sensing arrangement may further comprise a corresponding Hall effect detector configured to detect a magnetic field intensity of the magnet. The loading angular position may be detected when the detected magnetic field intensity has reached a maximum. The Hall effect detector measures the magnetic field intensity in general. In particular, the measured signal will be maximum when the magnetic is closest to the detector when utilizing one magnet per rotatable gear.

In one alternative embodiment, the sensing arrangement comprises two magnets attached to the first gear or the second gear, and two magnets attached to the loading assembly, and a corresponding Hall effect detector. The Hall effect detector may be configured to detect a magnetic field intensity, wherein the loading angular position is detected when the detected magnetic field intensity crosses zero. In this embodiment, it is the aim to detect a so called zero crossing of the magnetic field intensity instead of the peaks of the magnetic field. Based on both methods, the loading angular position can be detected properly.

In a fourth aspect of the invention, a robotic module assembly for an endovascular procedure is provided. The robotic module assembly comprises a pathway for accommodating a tubular device for an endovascular procedure. The pathway extends along a longitudinal axis of the robotic module assembly. The robotic module assembly furthermore comprises a first drive roller arranged on a first side of the pathway and a second drive roller arranged on a second side of the pathway. The drive rollers are configured to advance and/or retract the tubular device. The robotic module assembly furthermore comprises a gear assembly comprising a slot. The slot extends in a longitudinal direction in parallel to the longitudinal axis through the gear assembly. The slot comprises an entry region having an opening at the outer radial surface of the gear assembly. Further, the gear assembly comprises teeth at the outer radial surface thereof. In the entry region, the slot is designed such that teeth arranged in the entry region are not completely replaced by the opening of the slot.

It has been found that by designing the slot such that teeth arranged in the entry region are not completely replaced by the opening of the slot, it can be assured that the teeth of the gear are permanently meshing with teeth of another gear.

In one embodiment, the slot follows a trajectory which is orthogonal to the longitudinal axis and is at least partially curved. Preferably, the slot is not aligned with the teeth in the entry region.

In another aspect of the present invention, a method for inserting a tubular device for an endovascular procedure into a robotic module assembly is presented. The method comprises providing a robotic module assembly as defined according to any one of the preceding embodiments and transferring the loading assembly of the robotic module assembly into a loading state, in which the pathway is accessible via the slot. The method further comprises loading the tubular device through the slot of the gear assembly into the pathway and transferring the loading assembly into a loaded state in which the pathway is not accessible via the slot. In the described way, the tubular device can be conveniently loaded into the pathway and is thereafter constrained so that the tubular device can be reliably moved.

In another aspect of the present invention, a method for removing a tubular device for an endovascular procedure from a robotic module assembly is presented. The method comprises providing a robotic assembly as defined by any one of the preceding embodiments and transferring the loading assembly of the robotic module assembly into a loading state, in which the pathway is accessible via the slot. The method furthermore comprises unloading the tubular device from the pathway through the slot of the gear assembly. The method ensures that the tubular device can be conveniently removed from a robotic module assembly.

In another aspect of the present invention, a method for detecting a loading angular position of a robotic module assembly is presented. The method comprises rotating the first gear and/or the second gear, detecting a magnetic field intensity caused by at least one magnet attached to the first gear and/or the second gear and the loading assembly, stopping the first gear and/or the second gear, when the detected magnetic field intensity has reached a maximum in case one magnet is attached to the corresponding gear and the loading assembly, or when the detected magnetic field intensity crosses zero, in case two magnets are attached to the corresponding gear and the loading assembly. The method allows to ensure that the first gear and the second gear are transferred into a loading angular position in which the tubular device can be conveniently inserted into the robotic module assembly.

In another aspect of the present invention, a robotic apparatus, in particular an endovascular robotic apparatus is presented. The robotic apparatus comprises a robotic module assembly as defined by any one of claims 1-12.

In another aspect of the present invention, a use of a robotic module assembly is presented. In particular, a use of a robotic module assembly as defined by any one of claims 1-12 for advancing and/or retracting a tubular device, in particular a guidewire, a catheter and/or a microcatheter is presented.

It shall be understood that the robotic module assembly of claim 1, the method of claim 13, the robotic apparatus of claim 14 and the use of claim 15 have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a robotic module assembly for an endovascular procedure in a first operating position,
Fig. 2 shows schematically and exemplarily the embodiment of a robotic module assembly according to Fig. 1 in a second operating position,
Fig. 3 shows schematically and exemplarily an alternative embodiment of a robotic module assembly for an endovascular procedure in a first operating position,
Fig. 4 shows schematically and exemplarily the embodiment of Fig. 3 in a second operating position,
Fig. 5 shows schematically and exemplarily a further alternative embodiment of a robotic module assembly for an endovascular procedure in a first operating position,
Fig. 6 shows schematically and exemplarily the embodiment of Fig. 5 in a second operating position,
Fig. 7 shows schematically and exemplarily an embodiment of a robotic apparatus,
Fig. 8 shows schematically and exemplarily an alternative embodiment of a robotic module assembly.

### DETAILED DESCRIPTION OF EMBODIMENTS

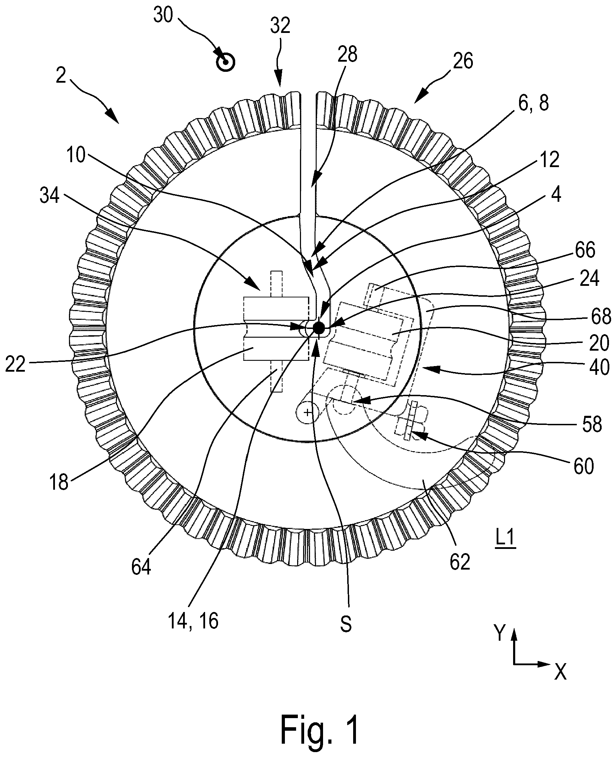

Fig. 1 shows a robotic module assembly 2 for an endovascular procedure. The robotic module 2 comprises a pathway 4 for accommodating a tubular device 14 for an endovascular procedure. The pathway 4 extends along a longitudinal axis 16 of the robotic module assembly 2. The robotic module assembly 2 comprises a first drive roller 18. The first drive roller 18 is arranged on a first side 22 of the pathway 4. The robotic module assembly 2 furthermore comprises a second drive roller 20 arranged on a second side 24 of the pathway 4. The drive rollers 18, 20 are configured to advance and/or retract the tubular device 14. The robotic module assembly 2 furthermore comprises a gear assembly 26. The gear assembly 26 comprises a slot 28 that extends in a longitudinal direction 30 in parallel to the longitudinal axis 16 through the gear assembly 26. The slot 28 is configured to load a tubular device 14 into the pathway 4.

The slot 28 extends from an outer radial surface 32 of the gear assembly 26 to the pathway 4 along a trajectory 6. The trajectory 6 is orthogonal to the longitudinal axis 16 of the robotic module assembly 2. In particular, the trajectory 6 is at least partially curved. The slot 28 comprises a transversal end portion 10. The transversal end portion 10 is aligned with the pathway 4. The transversal end portion 10 extends substantially parallel to the longitudinal axis 16 of the robotic module assembly 2. The transversal end portion 10 defines a sitting position for the tubular device 14 at the pathway 4. Adjacent to the transversal end portion 10, a transversal curved intermediate portion 12 is arranged. The curved intermediate portion 12 defines a turn of the tubular device 14. The curved intermediate portion 12 is designed such that the slot trajectory does not intersect the first drive roller 18. The tubular device 14 passes the transversal curved intermediate portion 12 before reaching the transversal end portion 10 when the tubular device 14 is inserted into the slot 28 towards the pathway 4. The tubular device 14 may be a guidewire, a catheter and/or a microcatheter.

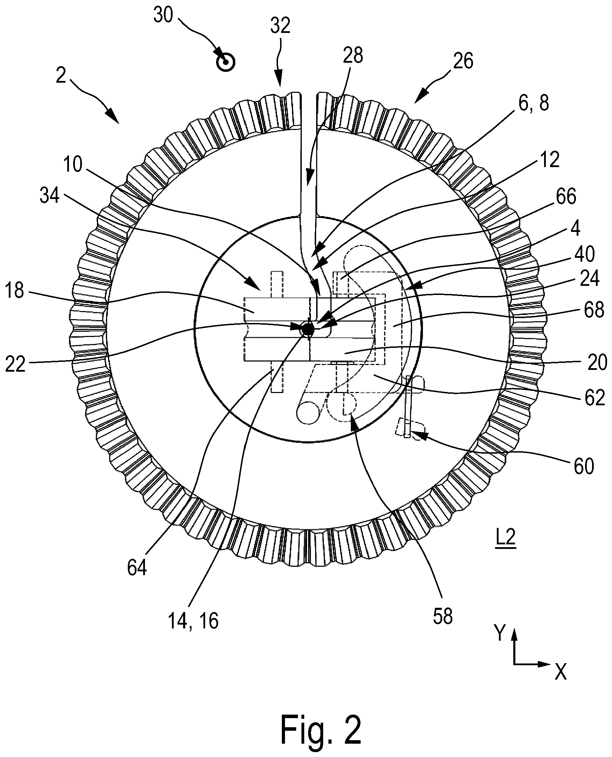

The robotic module assembly 2 furthermore comprises a loading assembly 34. The loading assembly 34 is configured to modify a spacing s between the first drive roller 18 and the second drive roller 20 between a loading state L1 and a loaded state L2. The loading state L1 is shown in Fig. 1. The loaded state L2 is shown in Fig. 2. The spacing s of the drive rollers 18, 20 in the loading state L1 leaves the pathway 4 accessible via the slot 28. The spacing s of the drive rollers 18, 20 in the loaded state L2 prevents the pathway 4 from being accessible via the slot 28. The spacing s between the drive rollers 18, 20 is larger in the loading state L1 than in the loaded state L2. The tubular device 14 may be engaged between the drive rollers 18, 20 in the loaded state L2 in order to advance and/or retract the tubular device 14. The first drive roller 18 is arranged opposite from the second drive roller 20 in the loaded state L2. As shown in Fig. 1, the loading assembly 34 is configured to pivot at least one of the drive rollers 18, 20, namely an embodiment of Figs 1 and 2 the drive roller 20, in order to modify the spacing s between the drive rollers 18, 20 between the loading state L1 and the loaded state L2.

The loading assembly 34 comprises a lever 62 and a keyed shaft 58. The lever 62 rotates the keyed shaft 58. The keyed shaft 58 supports the loading assembly 34 in the loaded position L2 like a shim. The loading assembly 34 moreover comprises an elastic member 60. The elastic member 60 maintains the position with friction between the loading assembly 34 and the keyed shaft 58. The lever 62 is actuated to rotate the keyed shaft 58 into the loading state L1. In the loading state L1, the keyed shaft 58 no longer supports the loading assembly 34. The elastic member 60 rotates the loading assembly 34 such that a gap is formed between the first drive roller 18 and the second drive roller 20 as shown in Fig. 1.

The pathway 4 and the slot 28 constrain the tubular device 14 along a Y-axis. The drive rollers 18, 20 constrain the tubular device 14 along an X-axis. The first drive roller 18 comprises a first rotation axis 64. The second drive roller comprises a second rotation axis 66. The second rotation axis 66, together with the second drive roller 20, are accommodated in an accommodation structure 68.

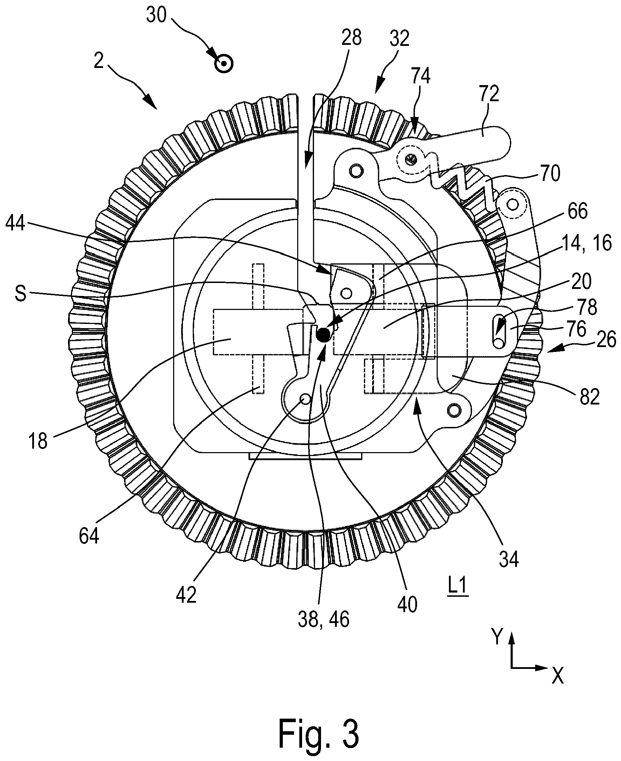

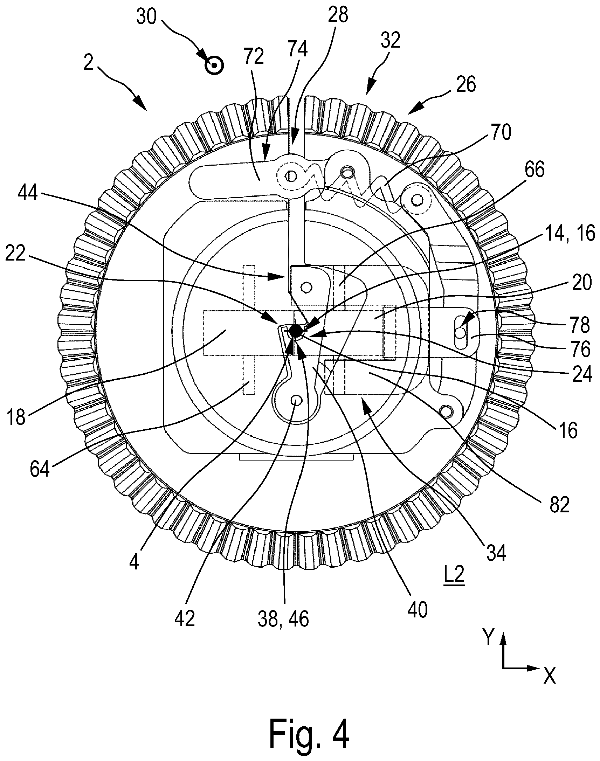

Figs 3 and 4 show an alternative embodiment of a robotic module assembly 2 for an endovascular procedure. The robotic module assembly 2 comprises a pathway 4 for accommodating a tubular device 14 for an endovascular procedure. The pathway 4 extends along a longitudinal axis 16 of the robotic module assembly 2. The robotic module assembly 2 furthermore comprises a first drive roller 18 arranged on a first side 22 of the pathway 4. The robotic module assembly 2 furthermore comprises a second drive roller 20 arranged on a second side 24 of the pathway 4.

The drive rollers 18, 20 are configured to advance and/or retract the tubular device 14. The robotic module assembly 2 comprises a slot 28 that extends in a longitudinal direction 30 in parallel to the longitudinal axis 16 through the gear assembly 26. The slot 28 is configured to load a tubular device 14 into the pathway 4. The slot 28 extends from an outer radial surface 32 of the gear assembly 26 to the pathway 4. The robotic module assembly 2 furthermore comprises a loading assembly 34. The loading assembly 34 is configured to modify a spacing s between the first drive roller 18 and the second drive roller 20 between a loading state L1 and a loaded state L2.

The loading assembly 34 comprises a sitting profile 38. The sitting profile 38 is configured to sit and/or accommodate the tubular device 14 therein. The sitting profile 38 matches the end of the slot 28 in the loading state L1. The position of the sitting profile 38 in the loading state L1 is different from the position of the sitting profile 38 in the loaded state L2. This is illustrated in the different operating positions shown in Figs 3 and 4. The loading assembly 34 comprises a pivotable portion 40. The sitting profile 38 is arranged in the pivotable portion 40.

The sitting profile 38 is transferred between the loading state L1 as shown in Fig. 3 and the loaded state L2 as shown in Fig. 4 by pivoting the pivotable portion 40 about a pivot axis 42. The pivot axis 42 is spaced from the longitudinal axis 16 of the robotic module assembly 2. The pivotable portion 40 comprises a protruding edge 44. The protruding edge 44 extends adjacent to the slot 28. The protruding edge 44 closes the sitting profile 38 in the loaded state L2. The slot 28 comprises a bottom section 46. The bottom section 46 is aligned with the drive rollers 18, 20.

The loading assembly 34 furthermore comprises an over center linkage 74 comprising an elastic member 70 and a lever 72. The over center linkage 74 has two stable positions, namely the loading state L1 and the loaded state L2. The lever 72 is actuated to slide the second drive roller 20 between the loading state L1 and the loaded state L2. The loading assembly 34 furthermore comprises a rigid member 76 comprising a slot 78. The rigid member 76 pushes the loading assembly 34 into the loading state L1. The elastic member 70 holds the loading assembly 34 in the loaded state L2. In the loaded state L2, the sitting profile 38 and the protruding edge 44 fully constrain the tubular device 14 along an X-axis and a Y-axis.

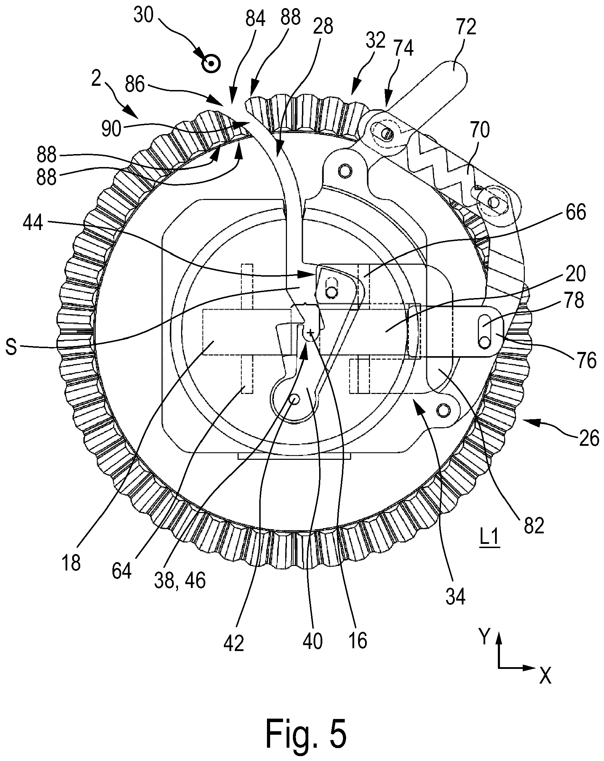

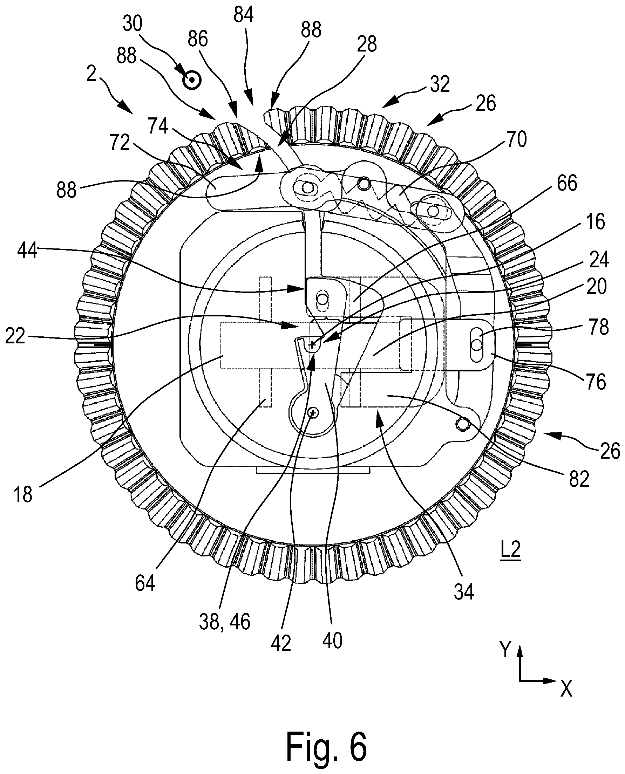

Figs 5 and 6 show an alternative embodiment of a robotic module assembly 2 for an endovascular procedure. The robotic module assembly 2 shown in Figs 5 and 6 is similar to the robotic module assembly 2 shown in Figs 3 and 4 but comprises a different geometry of the slot 28.

The robotic module assembly 2 comprises a pathway 4 for accommodating a tubular device 14 (not shown in Figs 5 and 6) for an endovascular procedure. The pathway 4 extends along a longitudinal axis 16 of the robotic module assembly 2. The robotic module assembly 2 furthermore comprises a first drive roller 18 arranged on a first side 22 of the pathway 4. The robotic module assembly 2 furthermore comprises a second drive roller 20 arranged on a second side 24 of the pathway 4.

The drive rollers 18, 20 are configured to advance and/or retract the tubular device 14. The robotic module assembly 2 comprises a slot 28 that extends in a longitudinal direction 30 in parallel to the longitudinal axis 16 through the gear assembly 26. The slot 28 is configured to load a tubular device 14 into the pathway 4. The slot 28 extends from an outer radial surface 32 of the gear assembly 26 to the pathway 4. The robotic module assembly 2 furthermore comprises a loading assembly 34. The loading assembly 34 is configured to modify a spacing s between the first drive roller 18 and the second drive roller 20 between a loading state L1 and a loaded state L2. The loading state L1 is shown in Fig. 5. For the loaded state L2, reference is made to Fig. 6.

The loading assembly 34 comprises a sitting profile 38. The sitting profile 38 is configured to sit and/or accommodate the tubular device 14 therein. The sitting profile 38 matches the end of the slot 28 in the loading state L1. The position of the sitting profile 38 in the loading state L1 is different from the position of the sitting profile 38 in the loaded state L2. The loading assembly 34 comprises a pivotable portion 40. The sitting profile 38 is arranged in the pivotable portion 40.

The sitting profile 38 is transferred between the loading state L1 as shown in Fig. 5 and the loaded state L2 as shown in Fig. 6 by pivoting the pivotable portion 40 about a pivot axis 42. The pivot axis 42 is spaced from the longitudinal axis 16 of the robotic module assembly 2. The pivotable portion 40 comprises a protruding edge 44. The protruding edge 44 extends adjacent to the slot 28. The protruding edge 44 closes the sitting profile 38 in the loaded state L2. The slot 28 comprises a bottom section 46. The bottom section 46 is aligned with the drive rollers 18, 20.

The loading assembly 34 furthermore comprises an over center linkage 74 comprising an elastic member 70 and a lever 72. The over center linkage 74 has two stable positions, namely the loading state L1 and the loaded state L2. The lever 72 is actuated to slide the second drive roller 20 between the loading state L1 and the loaded state L2. The loading assembly 34 furthermore comprises a rigid member 76 comprising a slot 78. The rigid member 76 pushes the loading assembly 34 into the loading state L1. The elastic member 70 holds the loading assembly 34 in the loaded state L2. In the loaded state L2, the sitting profile 38 and the protruding edge 44 fully constrain the tubular device 14 along an X-axis and a Y-axis.

The slot 28 comprises an entry region 84 having an opening 86 at the outer radial surface 32 of the gear assembly 26. Further, the gear assembly 26 comprises teeth 88 at the outer radial surface 32. In the entry region 84, the slot 28 is designed such that teeth 88 arranged in the entry region 84 are not completely replaced by the opening 86 of the slot 28. The slot 28 follows a trajectory 90 which is at least partially curved and orthogonal to the longitudinal axis 16. The slot 28 is not aligned with the teeth 88 in the entry region 84.

By designing the slot 28 such that teeth 88 arranged in the entry region 84 are not completely replaced by the opening 86 of the slot 28, it can be assured that the teeth 88 of the gear assembly 26 are permanently meshing with teeth of another gear (not shown).

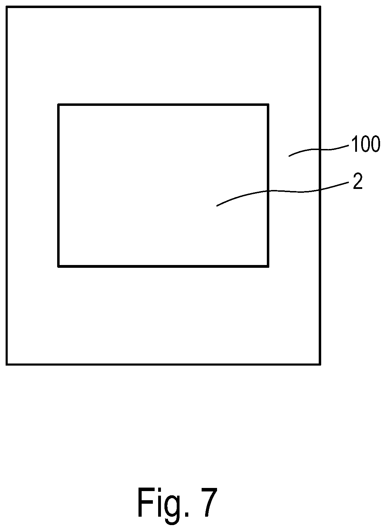

Fig. 7 shows a robotic apparatus 100. The robotic apparatus 100 comprises a robotic module assembly 2 as shown in any one of Figs 1-6.

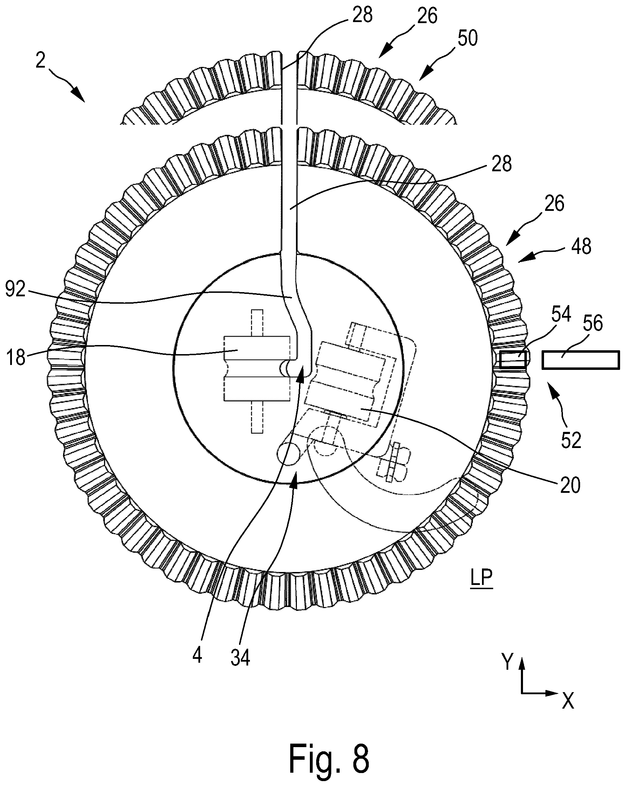

Fig. 8 shows a robotic module assembly 2 similar to the one shown in Figs 1 and 2. The gear assembly 26 comprises a first rotatable gear 48 and a second rotatable gear 50, which is shown only partially and schematically. The gears 48, 50 are arranged substantially in parallel to each other. Each gear 48, 50 comprises a slot 28. The slots 28 are configured to load a tubular device 14 into the pathway 4 when the slots 28 of the two gears 48, 50, and the loading assembly 34 are aligned in a loading angular position LP.

The gear assembly 26 comprises a sensing arrangement 52. The sensing arrangement 52 is configured to detect the loading angular position LP of the first rotatable gear 48 and the second rotatable gear 50. The sensing arrangement 52 comprises a magnet 54. The magnet 54 may be attached to the first gear 48 and/or second gear 50 and also the loading assembly 34 (not shown in Fig. 8). The sensing arrangement 52 comprises a Hall effect detector 56. The Hall effect detector 56 is configured to detect a magnetic field intensity of the magnet 54. The loading angular position LP is detected when the detected magnetic field intensity has reached a maximum.

The sensing arrangement 52 may alternatively comprise two magnets 54 (not shown) wherein two magnets 54 are attached to the first gear 48 and/or second gear 50 and also the loading assembly 34. The sensing arrangement 52 may comprise the Hall effect detector 56 configured to detect a magnetic field intensity. For this configuration, the loading angular position LP is detected when the detected magnetic field intensity crosses zero.

During operation, the first gear 48 and/or the second gear 50 may be rotated. During rotation, a magnetic field intensity caused by at least one magnet 54 attached to the first gear 48 and the loading assembly 34 is detected or measured. The first gear 48 and/or the second gear 50 may be stopped, when the detected magnetic field intensity has reached a maximum in case one magnet 54 is attached to the gear 48 and/or gear 50 and one magnet 54 is attached to loading assembly 34, or when the detected magnetic field intensity crosses zero, in case two magnets 54 are attached to the gear 48 and/or gear 50 and two magnets 54 are attached to loading assembly 34.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like detecting a magnetic field intensity caused by at least one magnet, et cetera, performed by one or several units or devices can be performed by any other number of units or devices. These procedures can be implemented as program code means of a computer program and/or as dedicated hardware.

Any reference signs in the claims should not be construed as limiting the scope.

## Claims

1. Robotic module assembly (2) for an endovascular procedure, the robotic module assembly (2) comprising:
- a pathway (4) for accommodating a tubular device (14) for an endovascular procedure, the pathway (4) extending along a longitudinal axis (16) of the robotic module assembly (2),
- a first drive roller (18) arranged on a first side (22) of the pathway (4) and a second drive roller (20) arranged on a second side (24) of the pathway (4), wherein the drive rollers (18, 20) are configured to advance and/or retract the tubular device (14),
- a gear assembly (26) comprising a slot (28) that extends in a longitudinal direction (30) in parallel to the longitudinal axis (16) through the gear assembly (26), wherein the slot (28) is configured to load a tubular device (14) into the pathway (4), wherein the slot (28) extends from an outer radial surface (32) of the gear assembly (26) to the pathway (4) along a trajectory (6), and wherein the trajectory (6) is at least partially curved.

2. The robotic module assembly (2) as defined by claim 1, wherein the slot (28) comprises a transversal end portion (10) aligned with the pathway (4), wherein the transversal end portion (10) extends substantially parallel to the longitudinal axis (16) of the robotic module assembly (2), and wherein the end portion (10) defines a sitting position for the tubular device (14) at the pathway (4).

3. The robotic module assembly (2) as defined by claim 2, wherein adjacent to the transversal end portion (10) a transversal curved intermediate portion (12) is arranged, wherein the curved intermediate portion (12) defines a turn of the tubular device (14), and wherein the tubular device (14) passes the transversal curved intermediate portion (12) before reaching the transversal end portion (10) when the tubular device (14) is inserted into the slot (28) towards the pathway (4).

4. The robotic module assembly (2) as defined by any one of the preceding claims, further comprising a loading assembly (34) configured to modify a spacing (s) between the first drive roller (18) and the second drive roller (20) between a loading state (L1) and a loaded state (L2), wherein the spacing (s) of the drive rollers (18, 20) in the loading state (L1) leaves the pathway (4) accessible via the slot (28) and wherein the spacing (s) of the drive rollers in the loaded state (L2) prevents the pathway (4) from being accessible via the slot (28).

5. The robotic module assembly (2) as defined by claim 4, wherein the loading assembly (34) is configured to pivot at least one of the drive rollers (18, 20) in order to modify the spacing (s) between the drive rollers (18, 20) between the loading state (L1) and the loaded state (L2).

6. The robotic module assembly (2) as defined by any one of claims 4 or 5, wherein the loading assembly (34) comprises a sitting profile (38) configured to sit and/or accommodate the tubular device (14) therein.

7. The robotic module assembly (2) as defined by claim 6, wherein the sitting profile (38) matches the end of the slot (28) in the loading state (L1).

8. The robotic module assembly (2) as defined by claim 7, wherein the loading assembly (34) comprises a pivotable portion (40), in which the sitting profile (38) is arranged, and wherein the sitting profile (38) is transferred between the loading state (L1) and the loaded state (L2) by pivoting the pivotable portion (40) about a pivot axis (42).

9. The robotic module assembly (2) as defined by any one of claims 4 to 8, wherein the gear assembly (26) comprises a first rotatable gear (48) and a second rotatable gear (50), wherein the gears (48, 50) are arranged substantially in parallel to each other, and wherein each gear (48, 50) comprises said slot (28), and wherein the loading assembly comprises a slot (92), wherein the slots (28, 92) are configured to load a tubular device (14) into the pathway (4) when the slots (28) are aligned in a loading angular position (LP).

10. The robotic module assembly (2) as defined by claim 9, wherein the gear assembly (26) comprises a sensing arrangement (52) configured to detect the loading angular position (LP) of the first rotatable gear (48) and/or the second rotatable gear (50).

11. The robotic module assembly (2) as defined by claim 10, wherein the sensing arrangement (52) is configured to detect the loading angular position (LP) of the first rotatable gear (48), the second rotatable gear (50), and the loading assembly (34).

12. The robotic module assembly (2) as defined by any one of claims 10 or 11, wherein the sensing arrangement (52) comprises a magnet (54) attached to the first gear (48) and/or the second gear (50), and a magnet (54) attached to the loading assembly (34), and a corresponding Hall effect detector (56) configured to detect a magnetic field intensity of the magnet (54), wherein the loading angular position (LP) is detected when the detected magnetic field intensity has reached a maximum,
or wherein the sensing arrangement (52) comprises two magnets (54) attached to the first gear (48) and/or the second gear (50), and two magnets (54) attached to the loading assembly (34), and a corresponding Hall effect detector (56) configured to detect a magnetic field intensity, wherein the loading angular position (LP) is detected when the detected magnetic field intensity crosses zero.

13. Method for detecting a loading angular position (LP) of a robotic module assembly (2) as defined by claim 12, the method comprising:
- rotating the first gear (48) and/or the second gear (50),
- detecting a magnetic field intensity caused by at least one magnet (54) attached to the first gear (48) and/or the second gear (50) and the loading assembly (34),
- stopping the first gear (48) and/or the second gear (50), when the detected magnetic field intensity has reached a maximum in case one magnet (54) is attached to the corresponding gear (48, 50) and the loading assembly (34), or when the detected magnetic field intensity crosses zero, in case two magnets (54) are attached to the corresponding gear (48, 50) and the loading assembly (34).

14. Robotic apparatus (100), in particular endovascular robotic apparatus, the apparatus comprising a robotic module assembly (2) as defined by any one of claims 1-12.

15. Use of a robotic module assembly (2) as defined by any one of claims 1-12, for advancing and/or retracting a tubular device (14), in particular a guidewire, a catheter and/or a microcatheter.
